(19) Europäisches Patentamt
European Patent Office
Office européen des brevets

(11) Numéro de publication: **0 584 315 B1**

(12) # FASCICULE DE BREVET EUROPEEN

(45) Date de publication du fascicule du brevet: **20.09.95**

(51) Int. Cl.$^{6}$: **A61K 7/00**, A61K 9/127

(21) Numéro de dépôt: **93904163.8**

(22) Date de dépôt: **08.02.93**

(86) Numéro de dépôt internationale :
**PCT/FR93/00128**

(87) Numéro de publication internationale :
**WO 93/15708 (19.08.93 93/20)**

(54) **COMPOSITION COSMETIOUE ET/OU PHARMACEUTIOUE CONTENANT UNE DISPERSION DE VESICULES LIPIDIOUES, PROCEDE DE PREPARATION DE LADITE DISPERSION ET DISPERSION DE VESICULES LIPIDIOUES.**

(30) Priorité: **18.02.92 FR 9201821**

(43) Date de publication de la demande:
**02.03.94 Bulletin 94/09**

(45) Mention de la délivrance du brevet:
**20.09.95 Bulletin 95/38**

(84) Etats contractants désignés:
**AT BE CH DE DK ES FR GB GR IE IT LI NL PT SE**

(56) Documents cités:
**EP-A- 0 154 977**
**EP-A- 0 287 876**
**EP-A- 0 343 444**
**EP-A- 0 422 978**
**EP-A- 0 423 011**
**EP-A- 0 444 983**
**WO-A-87/06460**
**DE-A- 1 149 700**
**GB-A- 2 189 457**

(73) Titulaire: **L'OREAL**
**14, rue Royale**
**F-75008 Paris (FR)**

(72) Inventeur: **MORANCAIS, Jean-Luc**
**11, rue Georges-Brassens**
**F-77330 Ozoir-la-Ferrière (FR)**
Inventeur: **LETY, Alain**
**9, rue de Metz**
**F-77400 Lagny-sur-Marne (FR)**
Inventeur: **VANLERBERGHE, Guy**
**40, rue du Général-de-Gaulle,**
**Montjay la Tour**
**F-77410 Villevaude (FR)**

(74) Mandataire: **Peuscet, Jacques**
**SCP Cabinet Peuscet et Autres,**
**68, rue d'Hauteville**
**F-75010 Paris (FR)**

EP 0 584 315 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

BIOCHEM. BIOPHYS. ACTA, LIPIDS AND LIPID METABOLISM vol. 1047, no. 1, 1990, pages 1 - 10 YUE ,YUNSHI ET AL. 'DEUTERIUM NMR STUDY OF THE INTERACTION OF PHYTANIC ACID AND PHYTOL WITH THE HEAD GROUP REGION OF A PHOSPHOLIPID BILAYER. EVIDENCE OF MAGNETIC ORIENTATION.'

CAN. J. CHEM. vol. 55, no. 2, 1977, pages 220 - 226 CUSHLEY ET AL. 'STRUCTURE AND STABILITY OF VITAMIN E - LECITHIN AND PHYTANIC ACID - LECITHIN BILAYERS STUDIED BY 13C AND 31P NUCLEAR MAGNETIC RESONANCE" cité dans la demande

## Description

La présente invention concerne une composition cosmétique et/ou pharmaceutique contenant une dispersion de vésicules de lipides amphiphiles ioniques et/ou non-ioniques transparente, un procédé de préparation de ladite dispersion et une dispersion utilisée dans ladite composition.

On sait qu'il est possible de préparer des vésicules à partir de certains lipides amphiphiles, c'est-à-dire de molécules constituées d'une partie lipophile et d'une partie hydrophile. Les vésicules obtenues sont délimitées par une membrane de phase lipidique formée d'un feuillet ou de plusieurs feuillets concentriques, ladite membrane définissant un volume interne fermé où est encapsulée une phase, dite phase encapsulée. Les vésicules sont généralement préparées sous forme de dispersion dans une phase aqueuse, dite phase de dispersion. Les lipides amphiphiles peuvent être des lipides ioniques tels que les lécithines naturelles (lécithine d'oeuf, de soja) ou synthétiques (dipalmitoylécithine, lécithine d'oeuf hydrogéné). Les lipides amphiphiles peuvent également être des lipides non-ioniques tels que des dérivés de polyglycérol linéaires ou ramifiés, des éthers de polyglycérol linéaires ou ramifiés, des alcools gras polyoxyéthylénés, des stérols polyoxyléthylénés, des éthers de polyols, des esters de polyols oxyéthylénés ou non, des glycolipides, certains hydroxyamides et autres.

La phase lipidique peut contenir un ou plusieurs lipides amphiphiles ioniques, un ou plusieurs lipides amphiphiles non-ioniques ou, à la fois au moins un lipide amphiphile ionique et au moins un lipide amphiphile non-ionique.

Il est connu d'introduire dans la phase lipidique au moins un principe cosmétiquement et/ou pharmaceutiquement actif lipophile, la nature et la quantité des réactifs introduits étant choisies de façon à ne pas détériorer la stabilité des vésicules. On peut également, de façon connue, introduire des actifs hydrophiles dans la phase aqueuse encapsulée et/ou dans la phase aqueuse de dispersion.

Pour des raisons de présentation, on cherche à préparer des dispersions de vésicules qui soient sensiblement transparentes, car les compositions cosmétiques préparées à partir de dispersions transparentes ont un aspect plus agréable et plus attractif pour l'utilisateur.

La transparence (ou à l'inverse, l'opacité) d'une dispersion, vis-à-vis de la lumière naturelle, est essentiellement fonction de l'indice de réfraction du milieu de dispersion et de celui des particules dispersées, de la concentration, de la taille moyenne et de l'homogénéité en taille de ces particules. Il est notamment possible d'augmenter la transparence d'une dispersion en diminuant la différence entre l'indice de réfraction des particules dispersées et celui du milieu de dispersion et/ou en diminuant la concentration et/ou la dimension des particules dispersées. On peut, bien entendu, améliorer la transparence d'une dispersion de vésicules lipidiques en diluant cette dispersion mais on diminuerait ainsi la concentration en principes actifs de la dispersion, ce qui doit généralement être évité. On a également essayé de réduire la dimension moyenne des vésicules, mais il est alors nécessaire d[1]utiliser pendant un temps trop important, lors de la préparation des vésicules, des moyens mécaniques puissants tels que des homogénéisateurs sous pression ou des sondes à ultrasons, ce qui conduit à une élévation du prix de revient. On a également songé à diminuer la polydispersité en utilisant des méthodes de classification telles que filtration sous pression ou fractionnement par taille à l'aide de colonnes de chromatographie : mais ces méthodes sont contraignantes et coûteuses.

Selon la présente invention, on a trouvé que l'on améliore considérablement la transparence de dispersions de vésicules dont la phase lipidique contient au moins un lipide amphiphile ionique et/ou au moins un lipide amphiphile non-ionique en introduisant dans ladite phase lipidique au moins un polyol ou un acide isoprénoïque à chaîne phytyle, sans qu'il soit nécessaire de modifier le mode de préparation des vésicules. On a constaté que même des dispersions ayant une forte concentration en lipides, par exemple une concentration supérieure à 5 % en poids, sont alors sensiblement transparentes.

La présente invention a donc pour premier objet une composition cosmétique et/ou pharmaceutique contenant au moins une dispersion, dans une phase aqueuse de dispersion, de vésicules délimitées par une membrane de phase lipidique contenant au moins un lipide amphiphile ionique et/ou au moins un lipide amphiphile non-ionique, lesdites vésicules contenant une phase encapsulée, caractérisée par le fait que, dans au moins une dispersion, la phase lipidique contient au moins un composé de formule

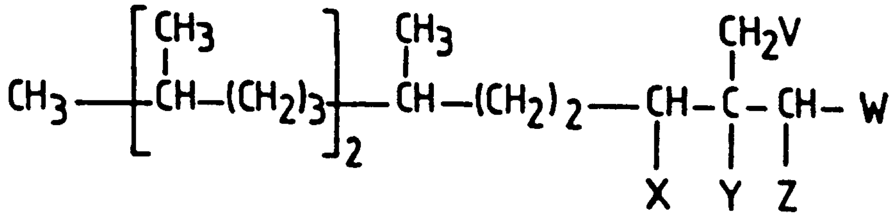

(I)

formule (I) dans laquelle :

- ou bien W représente $-CH_2OH$, -COOM ou $-(CH_2)_2$-COOM, où M représente H, un métal alcalin ou un métal alcalinoterreux, auquel cas X, Y, Z, V, identiques ou différents, représentent un atome d'hydrogène ou un radical hydroxyle, avec la condition que, lorsque W représente le groupement $-CH_2OH$, au moins un des radicaux X, Y, Z ou V représente un radical hydroxyle;
- ou bien W représente le groupement $-CH_3$, auquel cas X, Y, Z et V représentent H ou -OH combinés comme indiqué dans chaque ligne du tableau ci-dessous :

| X | Y | Z | V |
|---|---|---|---|
| OH | OH | OH | OH |
| OH | H | OH | OH |
| OH | OH | OH | H |
| H | OH | OH | OH |
| H | OH | OH | H |
| H | H | OH | OH |
| OH | H | OH | H |

Les composés de formule (I), que l'on préfère, sont le phytanetriol ou 3,7,11,15-tétraméthyl 1,2,3-trihydroxy hexadécane, l'acide 5,9,13,17 -tétraméthyl octadécanoïque, et les tétrols tels que le 3,7,11,15 -tétraméthyl 1,2,3,4-tétrahydroxy hexadécane ou le 3-hydroxyméthyl 7,11,15-triméthyl 1,2,4-trihydroxy hexadécane obtenus comme sous-produits de la synthèse du phytanetriol par oxydation du phytol à l'eau oxygénée.

Comme expliqué ci-dessus, le premier avantage des dispersions de la composition selon l'invention est que ces dispersions sont transparentes même pour de fortes concentrations en lipides.

On a constaté, par ailleurs, que, dans le cas de la préparation de certaines vésicules, l'introduction d'au moins un composé de formule (I) dans la phase lipidique permettait de diminuer ou de supprimer le cholestérol, qui est couramment utilisé comme lipide constitutif de la paroi des vésicules, dans le but de stabiliser les vésicules. Le remplacement du cholestérol par un (des) composé(s) de formule (I) présente un intérêt sensible car les composés de formule (I) sont très difficilement oxydables, alors que le cholestérol s'oxyde relativement facilement en donnant des produits d'oxydation indésirables. En outre, les dispersions utilisées selon l'invention sont généralement obtenues directement avec un faible indice de polydispersité sans qu'il soit nécessaire, au cours de leur préparation, de recourir à un fractionnement onéreux. Enfin, on a constaté que l'application topique de certaines des dispersions permet de diminuer le module d'élasticité du stratum corneum ce qui présente un intérêt tout particulier en cosmétique.

Les composés de formule (I) sont des composés connus (voir par exemple "Progress in the chemistry of fats and other lipids" Volume XIV, Part 1, pages 5 à 44, Ak Lough, Editeur Ralph T Holman, Pergamon Press). Il est connu par un article de YUE et coll. (Biochimica et Biophysica Acta, Vol. 1047 n° 1 pages 1-102) de préparer une dispersion de vésicules à partir d'une phase lipidique contenant des phospholipides, en particulier la dipalmitoyl phosphatidylcholine et de l'acide phytanique, pour étudier l'influence de l'acide phytanique sur la couche de phospholipide, par résonance magnétique nucléaire. Cette étude montre que la présence d'acide phytanique dans la membrane vésiculaire provoque une réorientation des couches de phospholipide dans le champ magnétique. Selon un article de R. J. CUSHLEY et coll. (Can. J. of Chemistry Vol. 55 1977 pages 220-226), l'introduction d'acide phytanique dans la phase lipidique de vésicules de lécithine destabiliserait fortement la structure de la membrane vésiculaire. On a aussi proposé d'utiliser le

phytanetriol en cosmétique (voir CH-A 399 655, JP-A 63/5050 et JP-A 86/236 737).

Mais, jusqu'à ce jour, l'utilisation d'une dispersion vésiculaire contenant des composés de formule I dans des compositions cosmétiques et/ou pharmaceutiques n'a jamais été proposée et rien ne pouvait suggérer à l'homme de métier les avantages particuliers d'une telle utilisation, en particulier que les dispersions obtenues seraient transparentes.

Dans la phase lipidique constituant la membrane des vésicules, les composés de formule (I) représentent en poids de 5 à 90 % et, de préférence, de 10 à 60 % de la phase lipidique. Pour des quantités inférieures à 5 %, il n'y a pas d'amélioration notable de la transparence. Pour des quantités supérieures à 90 %, les vésicules obtenues ne sont plus assez stables.

La phase lipidique constitutive des membranes des vésicules de la dispersion selon l'invention peut comprendre, de façon connue, au moins un lipide choisi dans le groupe formé par :

A) les lipides non-ioniques ci-après définis :

(1) les dérivés du glycérol, linéaires ou ramifiés, de formule

$$R_0O-[C_3H_5(OH)O]_{\bar{n}}-H \qquad (II)$$

formule (II) dans laquelle :

- $-C_3H_5(OH)O-$
  est représenté par les structures suivantes prises en mélange ou séparément :
  $-CH_2CHOHCH_2O-$,

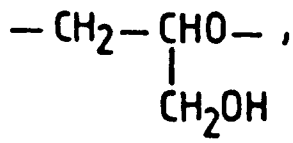 , 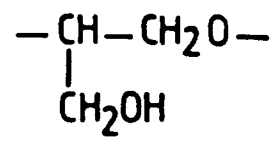

- $\bar{n}$ est une valeur statistique moyenne comprise entre 1 et 6 ou bien n = 1 ou 2 et alors $-C_3H_5-(OH)O-$ est représenté par la structure $-CH_2CHOH-CH_2O-$;
- $R_o$ représente :
  (a) une chaîne aliphatique, linéaire ou ramifiée saturée ou insaturée, contenant de 12 à 30 atomes de carbone ; ou des radicaux hydrocarbonés des alcools de lanoline ; ou les restes d'alpha-diols à longue chaîne;
  (b) un reste $R_1CO$, où $R_1$ est un radical aliphatique, linéaire ou ramifié, en $C_{11}-C_{29}$;
  (c) un reste

$$R_2-[OC_2H_3(R_3)]-$$

  où :
  - $R_2$ peut prendre la signification (a) ou (b) donnée pour $R_o$;
  - $-OC_2H_3(R_3)-$ est représenté par les structures suivantes, prises en mélange ou séparément :

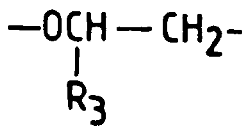 et 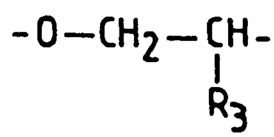

  où $R_3$ prend la signification (a) donnée pour $R_o$;

(2) les éthers de polyglycérol, linéaires ou ramifiés, comportant deux chaînes grasses ;

(3) les diols à chaîne grasse ;

(4) les alcools gras oxyéthylénés ou non, les stérols tels par exemple le cholestérol et les phytostérols, oxyéthylénés ou non;

(5) les éthers et esters de polyols, oxyéthylénés ou non, l'enchaînement des oxydes d'éthylène pouvant être linéaire ou cyclique ;

(6) les glycolipides d'origine naturelle ou synthétique, les éthers et esters de mono ou polysaccharides et notamment les éthers et les esters de glucose ;

(7) les hydroxyamides décrits dans le brevet français n° 2 588 256 et représentés par la formule :

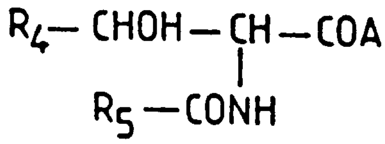

(III)

formule (III) dans laquelle :

- $R_4$ désigne un radical alkyle ou alcényle en $C_7$-$C_{21}$ ;
- $R_5$ désigne un radical hydrocarboné, saturé ou insaturé, en $C_7$-$C_{31}$ ;
- COA désigne un groupement choisi parmi les deux groupements suivants :
  - un reste

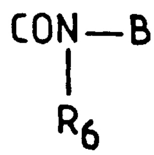

où :

- B est un radical alcoyle dérivé d'amines primaires ou secondaires, mono- ou polyhydroxylées ; et
- $R_6$ désigne un atome d'hydrogène ou un radical méthyle, éthyle ou hydroxyéthyle ; et
- un reste -COOZ, où Z représente le reste d'un polyol en $C_3$-$C_7$.

(8) les céramides naturels ou de synthèse ;

(9) les dihydroxyalkylamines, les amines grasses oxyéthylénées ;

(10) les dérivés du glycérol décrits dans la demande de brevet PCT n°91/00889 déposée le 13 Novembre 1991 et répondant à la formule :

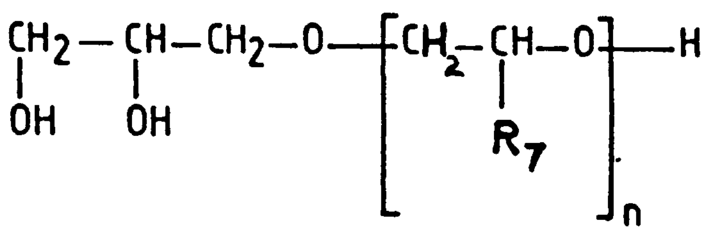

(IV)

formule (IV) dans laquelle $R_7$ représente un radical alkyle linéaire en $C_{14}$ à $C_{18}$ ou un groupement $-CH_2A$ dans lequel A est $-OR_{14}$, $R_{14}$ représentant un radical alkyle linéaire en $C_{10}$-$C_{18}$ et, de préférence, en $C_{16}$, et n représente une valeur statistique moyenne supérieure à 1 et au plus égale à 3 et, en outre, lorsque $R_7$ = $-CH_2A$, n peut également représenter une valeur réelle (non statistique) égale à 2.

B) les lipides amphiphiles ioniques ci-après définis :

(1) les lipides amphiphiles anioniques tels que :

- les phospholipides naturels, notamment la lécithine d'oeuf ou de soja, ou la sphingomyéline, les phospholipides modifiés par voie chimique ou enzymatique, notamment la lécithine hydrogénée,

et les phospholipides de synthèse, notamment la dipalmitoylphosphatidylcholine ;

- des composés anioniques, tels que ceux décrits dans le brevet français n° 2 588 256 et représentés par la formule

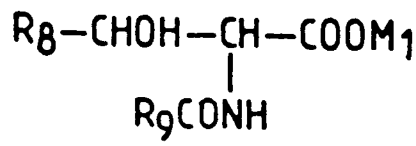

(V)

formule (V) dans laquelle :

- $R_8$ représente un radical alkyle ou alcényle en $C_7$-$C_{21}$,
- $R_9$ représente un radical hydrocarboné, saturé ou insaturé en $C_7$-$C_{31}$, et
- $M_1$ représente H, Na, K, $NH_4$ ou un ion ammonium substitué dérivé d'une amine,

(2) des composés anioniques, tels que les esters phosphoriques d'alcools gras, par exemple le dicétylphosphate et le dimyristylphosphate sous forme d'acides ou de sels alcalins ; l'acide heptylnonylbenzène sulfonique ; le sulfate de cholestérol acide et ses sels alcalins et le phosphate de cholestérol acide et ses sels alcalins ; les lysolécithines ; les alkylsulfates, par exemple le cétyl sulfate de sodium ; les gangliosides ;

(3) les lipides amphiphiles cationiques, tels que :

- les composés cationiques dérivés ammonium quaternaire répondant à la formule :

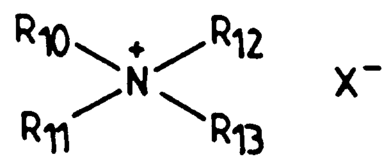

(VI)

avec $R_{10}$ et $R_{11}$, identiques ou différents, représentant des radicaux alkyle en $C_{12}$ - $C_{20}$ et $R_{12}$ et $R_{13}$, identiques ou différents, des radicaux alkyle en $C_1$ - $C_4$ ;

- les amines à longue chaîne et leurs dérivés ammonium quaternaire, les esters d'aminoalcools à longue chaîne et leurs sels et dérivés ammonium quaternaire ;
- des lipides polymérisables, comme ceux décrits par Ringsdorf et autres dans "Angewandte Chemie" vol 27, n° 1, Janvier 1988, pages 129 et 137.

Les lipides amphiphiles utilisés représentent ensemble, de préférence de 10 à 95 %, plus particulièrement de 40 à 90 %, du poids total de la phase lipidique vésiculaire.

On peut ajouter à la phase lipidique constituant la paroi des vésicules, des additifs non-lipidiques tels que certains polymères comme par exemple les polypeptides et les protéines.

La phase aqueuse de dispersion selon l'invention peut être constituée par de l'eau, ou un mélange d'eau et d'au moins un solvant miscible à l'eau tels que les alcools en $C_1$-$C_7$ et les polyols d'alkyle en $C_1$-$C_5$. La phase aqueuse de dispersion peut également contenir des composés en solution, tels que des sucres, des sels organiques ou minéraux ou des polymères dans la mesure où ils ne modifient pas ou peu la transparence de la dispersion.

La concentration en phase lipidique totale dans la dispersion est comprise entre 0,01 % et 50 % en poids, de préférence entre 1 % et 20 % en poids par rapport au poids total de la dispersion. Les vésicules dispersées ont des dimensions comprises entre 20 et 3000 nm, de préférence entre 20 et 500 nm.

Les compositions selon l'invention peuvent être utilisées même en l'absence de tout actif, comme produits cosmétiques pour le traitement de la peau. On a constaté notamment que, lorsque le composé de formule (I) est le phytanetriol, l'application topique de la composition permet de diminuer le module d'élasticité du stratum corneum.

Mais les compositions selon l'invention peuvent aussi contenir au moins un actif à action cosmétique et/ou pharmaceutique.

De façon connue, on peut introduire dans la phase lipidique des vésicules de la dispersion au moins un composé liposoluble cosmétiquement et/ou pharmaceutiquement actif. Selon l'invention, on peut utiliser tout actif liposoluble dans la mesure où il est compatible avec les composés de formule (I) utilisé(s) et où il ne modifie pas de façon gênante la transparence des compositions. Parmi ceux-ci, on peut citer à titre non

limitatif : l'acide rétinoïque, les lipoprotides et les stéroïdes.

La phase encapsulée dans les vésicules est généralement une phase aqueuse. De façon connue, on peut introduire dans la phase encapsulée et/ou dans la phase de dispersion des actifs hydrosolubles. De très nombreux composés de ce type ont été cités dans la littérature. Parmi ceux-ci, on peut citer à titre non limitatif : la glycérine, le sorbitol, l'érythrulose et les antibiotiques.

Des actifs amphiphiles peuvent aussi se répartir entre la phase aqueuse encapsulée et/ou de dispersion et la phase lipidique des vésicules.

Une liste des actifs utilisables dans les compositions selon l'invention est donnée dans le tableau I ci-après :

# TABLEAU I

| FONCTION | ACTIFS UTILISABLES |
|---|---|
| Anti-oxydant ou anti-radicaux libres. | Les extraits des plantes suivantes :<br>- Aubépine.<br>- Ginkgo biloba.<br>- Thé vert.<br>- Vigne.<br>- Romarin<br>Les enzymes :<br>- Commercialisées sous la dénomination SB 12 par SEDERMA et constituées par un mélange de lactoferrine et de lactoperoxydase, de glucose oxydase et de thiocyanate de potassium.<br>- Superoxyde dismutase.<br>- Glutathion peroxydase.<br>La superphycodismutase extraite d'algues.<br>Les coenzymes Q, en particulier la coenzyme Q10.<br>Les séquestrants, en particulier des dérivés d'acides polyphosphoniques.<br>Les tanins.<br>Le sélénium et ses dérivés, en particulier la séléniométhionine.<br>Les peptides, par exemple un mélange d'extraits de rate et de thymus,<br>Thiolim et sérum albumine bovine non stabilisée.<br>Les protéines, par exemple l'hémocyanine qui est une protéine cuivrée extraite de l'escargot marin et l'apohémocyanine qui est une protéine analogue sans cuivre.<br>Les flavonoïdes, notamment la catéchine, les proanthocyanidines, les flavanols, les flavones, les isoflavones, les flavanénols, les flavanones, les flavanes et les chalcones.<br>Les caroténoïdes, notamment le ß-carotène et le rocou.<br>L'acide sorbohydroxyamique.<br>Les tocophérols, notamment l'alpha-tocophérol et l'acétate d'alpha-tocophérol.<br>Le palmitate d'ascorbyle.<br>Le gallate de propyle.<br>L'acide caféique et ses dérivés.<br>L'acide ascorbique.<br>L'acide homogentisique.<br>L'acide érythorbique.<br>L'acide nordihydroguaïacétique.<br>Le laurylméthionate de lysine.<br>Le butylhydroxyanisole.<br>Le butylhydroxytoluène.<br>Les substances "SOD like". |

# TABLEAU I (suite 1)

| FONCTION | ACTIFS UTILISABLES |
|---|---|
| Hydratant ou humectant | Une reconstitution de sueur ("Normal moisturizing factors"-NMF).<br>Le pyroglutamate de sodium.<br>L'acide hyaluronique.<br>Les dérivés de chitosane (carboxyméthylchitine).<br>Le ß-glycérophosphate.<br>Le lactamide.<br>L'acétamide.<br>Le lactate d'éthyle, de sodium et de triéthanolamine.<br>Le pyrrolidone carboxylate de métaux en particulier de Mg, Zn, Fe, Ca ou Na.<br>La thiamorpholinone.<br>L'acide orotique.<br>Les acides carboxyliques alpha-hydroxylés en $C_3$ à $C_{20}$, notamment l'acide alpha-hydroxy propionique.<br>Les polyols, notamment l'inositol, le glycérol, la diglycérine, le sorbitol.<br>Les polyolosides, notamment alginate et guar.<br>Les protéines, notamment le collagène soluble et la gélatine.<br>Les lipoprotides choisis parmi les dérivés mono ou polyacylés d'acides aminés ou de polypeptides dans lesquels le reste acide RCO comporte une chaîne hydrocarbonée en $C_{13}$-$C_{19}$, notamment l'acide palmitoylcaséinique, l'acide palmitoyl collagénique, le dérivé dipalmitoyl-O-N de l'hydroxyproline, le stéaroyl glutamate de sodium, le stéaroyl tripeptide de collagène, l'oléyltétra et pentapeptide de collagène, le linoléate d'hydroxyproline.<br>L'urée et ses dérivés, notamment la méthylurée.<br>L'extrait de tissu cutané, notamment celui commercialisé sous la dénomination "OSMODYN" par les Laboratoires Serobiologiques de Nancy (LSN) et contenant des peptides, des acides aminés, des saccharides et 17% de mannitol.<br>Plus particulièrement une association de glycérol, d'urée et d'acide palmitoylcaséinique. |
| Mélanorégulateur :<br>1) accélérateur de bronzage | Les huiles de bergamote et de citrus.<br>L'alpha-MSH et ses homologues synthétiques.<br>La caféine.<br>Les dérivés de tyrosine, notamment le tyrosinate de glucose et la N-malyltyrosine. |

## TABLEAU I (suite 2)

| FONCTION | ACTIFS UTILISABLES |
|---|---|
| 2) Dépigmentant | L'acide ascorbique ou vitamine C et ses dérivés, notamment l'ascorbyle phosphate de Mg.<br>Les hydroxyacides, notamment l'acide glycolique.<br>L'acide kojique.<br>L'arbutine et ses dérivés.<br>L'hémocyanine (protéine cuivrée de l'escargot marin) et l'apohémocyanine (protéine analogue à la précédente sans cuivre).<br>L'hydroquinone et ses dérivés, notamment le monoalkyl éther et le benzyléther. |
| Coloration de la peau (brunissage artificiel) | L'ortho diacétylbenzène.<br>Les indoles.<br>La dihydroxyacétone.<br>L'érythrulose.<br>Le glycéraldéhyde.<br>Les gamma-dialdéhydes, notamment l'aldéhyde tartrique. |
| Liporégulateurs (amincissant et anti-acné, anti-séborrhée) | Les complexes de vitamines et d'oligo-éléments, notamment le complexe vitamine $B_6$/zinc.<br>L'orizanol.<br>L'acide azélaïque.<br>Les xanthines et alkylxanthines, notamment l'extrait de cola, la caféine et la théophylline.<br>L'adénosine monophosphate cyclique et non cyclique.<br>L'adénosine triphosphate.<br>L'extrait de lierre.<br>L'extrait de marron d'Inde.<br>Les extraits d'algues, notamment l'extrait d'algues rouges (fucus serratus) et le cytofiltrat.<br>L'extrait de ginseng.<br>L'extrait de Centella Asiatica (asiaticoside) contenant de la génine et de l'acide asiatique.<br>La thioxolone (HBT).<br>La S-carboxyméthylcystéine.<br>La S-benzylcystéamine. |

## TABLEAU I (suite 3)

| FONCTION | ACTIFS UTILISABLES |
|---|---|
| Antivieillissement et anti-rides | Les insaponifiables par exemple de soja et d'avocats.<br>Les acides gras insaturés, notamment l'acide linoléique et l'acide linolénique.<br>Les hydroxyacides, notamment l'acide glycolique.<br>Les facteurs de croissance.<br>Les complexes oligoéléments - vitamines, notamment $B_6$-Zn.<br>L'acide n-octanoyle-5 salicylique.<br>L'adénosine.<br>Le rétinol et ses dérivés, notamment l'acétate de rétinol et le palmitate de rétinol.<br>Les rétinoïdes, notamment les acides rétinoïques cis ou trans et ceux décrits dans les brevets FR-A-2 570 377 ; EP-A-199636 ; et EP-A- 325540 et la demande de brevet européen 90-402072.<br>L'association de rétinoïdes et de xanthines.<br>L'hydroxyproline.<br>Les acides sialiques.<br>L'extrait de rate, de thymus, Thiolim et sérum albumine bovine non stabilisé vendu sous la dénomination commerciale "SILAB" par la Société "SILAB".<br>Un extrait animal placentaire, notamment l'extrait embryonnaire placentaire de bovidés dans l'eau à 5,5 % stabilisé par 0,2 % d'exyl K100a (matrix).<br>Les protéoglycannes, en particulier le protéoglycanne de cartilage de trachée de bovidés à 5 % stabilisé (protéodermin).<br>Le colostrum.<br>Les facteurs d'oxygénation cellulaire, notamment l'octacosamol. |
| Anti-UV | Les filtres UV, notamment le paraméthoxycinnamate d'éthyl-2 hexyle ;<br>la benzophénone,<br>le benzylidène-camphre et leurs dérivés, en particulier, la tétrahydroxy-2,2', 4,4'-benzophénone et l'acide hydroxy-2 méthoxy-4 benzophénone-5 sulfonique ;<br>l'acide paraaminobenzoïque,<br>le salicylate de dipropylèneglycol,<br>l'octyl salicylate,<br>les dérivés de dibenzoylméthane vendus sous les marques EUSOLEX 8020 ou PARSOL 1789 et<br>les produits vendus sous les marques EUSOLEX 232, UNIVUL T 150, UNIVUL N 539, ESCALOL 507. |

## TABLEAU I (suite 4)

| FONCTION | ACTIFS UTILISABLES |
|---|---|
| Kératolytique | L'acide salicylique et ses dérivés, tels que les acides alkylsalicyliques, notamment l'acide n-octanoyl-5 salicylique et le n-dodécanoyl-5, salicylate de N-hexadécyl pyridinium.<br>L'acide rétinoïque.<br>Les enzymes protéolytiques, notamment la trypsine, l'alpha-chymotrypsine, la papaïne, la bromelaine et la pepsine.<br>Le peroxyde de benzoyle.<br>L'urée.<br>Les alpha-hydroxyacides. |
| Emollient | Esters tels que l'adipate d'isopropyle. |
| Anti-inflammatoire | Les corticoïdes tels que le 17-acétate de ß-méthasone, l'indométhacine, le ketoprofène, l'acide flufenamique, l'ibuprofene, le diclofenac, le diflunisal, le fenclofenac, le naproxene, le piroxicam, et le sulindac.<br>Le monostéaryl éther de glycérol (alcool batylique) et le mono-cétyléther de glycérol (alcool chimylique).<br>L'acide glycyrrhétinique et ses sels, notamment d'ammonium.<br>L'alpha-bisabolol (extrait de camomille).<br>La shikonine.<br>Des extraits de plantes, tels qu'eau de bleuet, arnica, aloès.<br>Des extraits de tissu méristématique, notamment l'extrait de racine de chêne.<br>Le plancton. |
| Rafraichissant | Le menthol.<br>Le lactate de menthyle. |
| Cicatrisant | L'arbre de peau, extrait de mimosa tenui flora.<br>L'extrait de Centella Asiatica.<br>L'acide ß-glycyrrhétinique.<br>L'hydroxyproline.<br>L'arginine.<br>Un extrait placentaire.<br>Un extrait de levure.<br>Le fagaramide.<br>La N-acétyl hydroxyproline.<br>L'acide acexamique et ses dérivés. |

TABLEAU I (suite 5)

| FONCTION | ACTIFS UTILISABLES |
|---|---|
| Protecteur vasculaire | Les flavonoïdes, notamment les dérivés de rutine, tels que l'étoxazorutine et le rutine propylsulfonate de sodium.<br>Les extraits végétaux, notamment l'extrait huileux de Ginkgo biloba, l'extrait de marron d'Inde (escine), de lierre (saponines) et de petit houx.<br>Le nicotinate d'alpha-tocophérol. |
| Anti-bactérien, antifongique | Le bromure de triméthylcétylammonium.<br>L'acide sorbique.<br>Le peroxyde de benzoyle.<br>Le chlorure de cétylpyridinium.<br>Le chlorure de benzalkonium.<br>L'acide parahydroxybenzoïque et ses sels.<br>Le bromo-2 nitro-2 propanediol-1,3.<br>Le trichloro-3,4,4'-carbanilide.<br>Le trichloro-2,4,4'-hydroxy-2 diphényléther.<br>L'acide déhydroacétique.<br>Un extrait de pamplemousse dans la glycérine et le propylène glycol.<br>La chlorhexidine.<br>L'hexetidine.<br>L'hexamidine. |
| Agent insectifuge | Le diméthyltoluamide. |
| Antiperspirant | Le chlorhydrate d'aluminium.<br>Le chlorure d'aluminium.<br>Le complexe lactate de sodium/aluminium chlorhydroxy.<br>Le chlorhydrate de zirconyle. |

## TABLEAU I (suite 6)

| FONCTION | ACTIFS UTILISABLES |
|---|---|
| Déodorant | L'oxyde de zinc.<br>Le ricinoléate de zinc.<br>L'éthyl-2 hexanediol-1,3.<br>L'hexachlorophène.<br>Le produit vendu sous la marque "IRGASAN DP 300". |
| Anti-pelliculaire | L'octopyrox.<br>Les omadines.<br>Le goudron de houille.<br>L'hydroxy-1 méthyl-4 triméthyle-2,4,4 pentyl-6 pyridinone-2.<br>Le sulfure de sélénium. |
| Anti-chute des cheveux | Les inhibiteurs de glucuronidases.<br>Les muccopolysaccharides.<br>Le nicotinate de méthyle ou d'hexyle.<br>La forskaline.<br>Le minoxidil.<br>Les xanthines.<br>Les rétinoïdes. |
| Colorant capillaire | Les bases et coupleurs d'oxydation.<br>Les colorants directs.<br>Les colorants auto-oxydables. |
| Agent décolorant pour cheveux | Le peroxyde d'hydrogène. |
| Réducteur pour permanentes | L'acide thioglycolique.<br>La cystéine.<br>La cystéamine.<br>La N-acétyl cystéine.<br>La N-acétyl cystéamine.<br>Le thioglycolate de glycérol. |
| Agent conditionneur pour peau et cheveux | Polymères cationiques, cations. |

Les dispersions contenues dans les compositions selon l'invention peuvent être préparées par tout mode de préparation connu pour la préparation des vésicules de lipide amphiphile. Divers modes de préparation sont, par exemple décrits dans "Les liposomes en biologie cellulaire et pharmacologie" Editions INSERM/John Libbey Emotext, 1987 pages 6 à 18.

Les dispersions de vésicules selon l'invention sont préparées, de préférence, par le procédé décrit ci-après :

- dans un premier stade, on prépare la phase lipidique devant former la membrane des vésicules par dissolution dans un solvant du (des) lipide(s) amphiphile(s), des composés de formule (I) et, éventuellement un (ou plusieurs) composé(s) cosmétiquement et/ou pharmaceutique-ment actif(s) liposoluble(s) et on évapore le solvant sous pression réduite ;
- dans un second stade, on ajoute la phase aqueuse de dispersion et on homogénéise le mélange par un moyen mécanique du type secouage et/ou ultrasons, pour obtenir la dispersion de vésicules.

L'homogénéisation est effectuée à une température comprise entre 10 °C et 120 °C, de préférence entre 30 et 80 °C.

Les compositions selon l'invention peuvent se présenter sous la forme de gels, de lotions ou de sérums en ajoutant, de façon connue, dans la phase aqueuse de dispersion des additifs de formulation n'ayant ni activité cosmétique, ni activité dermopharmaceutique propre. Parmi ces additifs, on peut citer les gélifiants, les polymères, les conservateurs, les colorants et les parfums.

La présente invention a également pour objet certaines dispersions de vésicules utilisées dans les compositions, selon l'invention. La présente invention a, par conséquent, pour objet les dispersions, dans une phase aqueuse de dispersion, de vésicules délimitées par une membrane de phase lipidique contenant au moins un lipide amphiphile ionique et/ou au moins un lipide amphiphile non-ionique, lesdites vésicules contenant une phase encapsulée, caractérisée par le fait que la phase lipidique contient au moins un composé de formule :

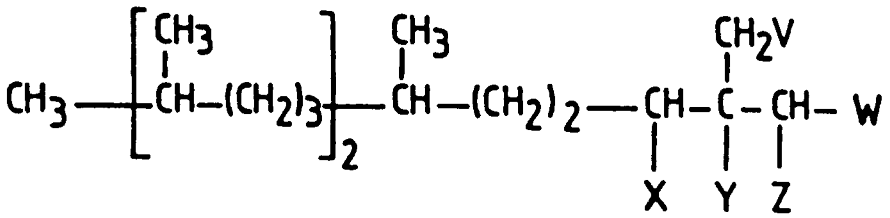

(I)

formule (I) dans laquelle :

- ou bien W représente $-CH_2OH$, -COOM ou $-(CH_2)_2-COOM$, où M représente H, un métal alcalin ou un métal alcalinoterreux, auquel cas X, Y, Z et V, identiques ou différents, représentent un atome d'hydrogène ou un radical hydroxyle, avec la condition que, lorsque W représente $-CH_2OH$, ou COOM, au moins un des radicaux X, Y, Z ou V représente un radical hydroxyle ;
- ou bien W représente un groupement $-CH_3$, auquel cas X, Y, Z et V représentent H ou -OH combinés comme indiqué dans chaque ligne du tableau ci-dessous :

| X | Y | Z | V |
|---|---|---|---|
| OH | OH | OH | OH |
| OH | H | OH | OH |
| OH | OH | OH | H |
| H | OH | OH | OH |
| H | OH | OH | H |
| H | H | OH | OH |
| OH | H | OH | H |

Une description plus détaillée de ces dispersions transparentes qui sont utilisées pour la fabrication de compositions cosmétiques et/ou pharmaceutiques a été donnée ci-dessus.

Pour mieux faire comprendre l'objet de l'invention, on va décrire maintenant, à titre d'exemples purement illustratifs et non limitatifs, plusieurs modes de mise en oeuvre.

Dans les exemples donnés ci-après, les dispersions ont été préparées de la façon suivante :
On introduit 1,5 g de phase lipidique ayant la composition donnée dans le tableau II ci-après dans un ballon

de 100 ml et on solubilise dans le solvant indiqué dans ledit tableau. Le solvant est ensuite évaporé à 40 °C par paliers successifs depuis la pression ambiante jusqu'à environ 500 Pa à l'aide d'un évaporateur rotatif.
Au film de phase lipidique obtenu, on ajoute 28,5 g de solution d'azoture de sodium ($NaN_3$) à 0,02 % en poids dans l'eau, pendant le temps et à la température donnés dans le tableau II ci-après.
Le mélange obtenu est agité à l'aide de la secoueuse à bras oscillants commercialisée par la société "PROLABO" sous la dénomination "OSCILL 12".
La dispersion obtenue, portée à la température de 30 °C est ensuite traitée pendant 2 mn à l'aide d'un homogénéisateur à ultrasons commercialisé par la société "BRANSON SONIC POWER Co" sous la dénomination "SONIFIER B 30" avec le réglage suivant :

- cycle de travail : 50 %
- réglage de puissance : position 5. Sur la dispersion obtenue, on a mesuré :
- la granulométrie, avec l'appareil commercialisé par la société "COULTER ELECTRONICS" sous la dénomination "COULTER N4", qui fonctionne sur le principe de la diffusion quasi-élastique de la lumière. La granulométrie des vésicules est caractérisée par un diamètre moyen (d) en nanomètres et un facteur de polydispersité en taille (Q). Ces deux paramètres sont calculés par la méthode des cumulants. Les mesures sont effectuées sur des dilutions contenant environ 0,3 % en poids de dispersion de vésicules.
- la densité optique des dispersions, à l'aide d'un spectrophotomètre UV-visible commercialisé par la société "BECKMAN" sous la dénomination "U.V. 5230", la mesure étant effectuée après dilution au quart de la dispersion à l'aide d'une solution d'azoture de sodium à 0,02 % en poids dans l'eau dans une cuve de 0,2 cm d'épaisseur, à 450 nm. Plus la densité optique $DO_{450}$ obtenue est faible, plus la dispersion est transparente.

Des essais comparatifs ont été effectués en l'absence de composé de formule (I) dans la phase lipidique et en présence d'hexadécanediol,1-2 qui est un polyol non ramifié ayant la même longueur de chaîne que les composés de formule (I).

Les résultats sont donnés dans le tableau II ci-après :

**TABLEAU II**

| Exemple N° | Composition de la phase lipidique | Conditions de préparation: Solvant utilisé dans préparation phase lipidique | Conditions de préparation: Agitation du mélange | d (nm) | Q polydispersité | $DO_{450}$ |
|---|---|---|---|---|---|---|
| 1* | lipide 1 ........ 0,825 g<br>cholestérol ........ 0,600 g<br>DCP ........ 0,075 g | 6,7 ml de dichlorométhane et 1,7 ml de méthanol | 2 heures à 45°C | 174 | 0,21 | 0,70 |
| 2 | lipide 1 ........ 0,825 g<br>cholestérol ........ 0,300 g<br>phytanetriol ........ 0,300 g<br>DCP ........ 0,075 g | 8,4 ml de dichlorométhane et 1,7 ml de méthanol | 2 heures à 45°C | 168 | 0,15 | 0,24 |
| 3* | lipide 1 ........ 0,825 g<br>cholestérol ........ 0,300 g<br>hexadecanediol 1-2 ..... 0,300 g<br>DCP ........ 0,075 g | 6,7 ml de dichlorométhane et 1,7 ml de méthanol | 2 heures à 45°C | 155 | 0,22 | 0,40 |

* ne fait pas partie de l'invention

## TABLEAU II (suite 1)

| Exemple N° | Composition de la phase lipidique | Conditions de préparation | | d (nm) | Q polydispersité | $DO_{450}$ |
|---|---|---|---|---|---|---|
| | | Solvant utilisé dans préparation phase lipidique | Agitation du mélange | | | |
| 4* | lipide 2 .................. 0,825 g<br>cholestérol .............. 0,600 g<br>DCP ..................... 0,075 g | 6,7 ml de chloroforme et 1,7 ml de méthanol | 2 heures à 70°C | 178 | 0,35 | 0,67 |
| 5 | lipide 2 .................. 0,825 g<br>cholestérol .............. 0,300 g<br>phytanetriol ............. 0,300 g<br>DCP ..................... 0,075 g | 6,7ml de chloroforme et 0,8 ml de méthanol | 2 heures à 70°C | 188 | 0,21 | 0,38 |
| 6* | lipide 2 .................. 0,825 g<br>cholestérol .............. 0,300 g<br>hexadecanediol 1-2 ..... 0,300 g<br>DCP ..................... 0,075 g | 6,7 ml de chloroforme et 1,7 ml de méthanol | 2 heures à 70°C | 177 | 0,25 | 0,69 |

* ne fait pas partie de l'invention

**TABLEAU II (suite 2)**

| Exemple N° | Composition de la phase lipidique | Conditions de préparation | | d (nm) | Q polydispersité | $DO_{450}$ |
|---|---|---|---|---|---|---|
| | | Solvant utilisé dans préparation phase lipidique | Agitation du mélange | | | |
| 7* | lipide 3 .................. 0,825 g<br>cholestérol .............. 0,600 g<br>DCP ..................... 0,075 g | 6,7 ml de dichlorométhane et 1,7 ml de méthanol | 2 heures à 70°C | 129 | 0,26 | 0,10 |
| 8 | lipide 3 .................. 0,825 g<br>cholestérol .............. 0,300 g<br>phytanetriol ............. 0,300 g<br>DCP ..................... 0,075 g | 6,7 ml de dichlorométhane et 1,7 ml de méthanol | 2 heures à 70°C | 104 | 0,28 | 0,05 |
| 9* | lipide 3 .................. 0,825 g<br>cholestérol .............. 0,300 g<br>hexadecanediol 1-2 ..... 0,300 g<br>DCP ..................... 0,075 g | 6,7 ml de dichlorométhane et 1,7 ml de méthanol | 2 heures à 70°C | 115 | 0,25 | 0,10 |

* ne fait pas partie de l'invention

**TABLEAU II (suite 3)**

| Exemple N° | Composition de la phase lipidique | Conditions de préparation | | d (nm) | Q polydispersité | $DO_{450}$ |
|---|---|---|---|---|---|---|
| | | Solvant utilisé dans préparation phase lipidique | Agitation du mélange | | | |
| 10* | lipide 4 ................... 0,713 g<br>cholestérol ............... 0,712 g<br>DCP ...................... 0,075 g | 6,7 ml de dichlorométhane | 2 heures à 70°C | 165 | 0,31 | 0,68 |
| 11 | lipide 4 ................... 0,713 g<br>cholestérol ............... 0,277 g<br>phytanetriol .............. 0,435 g<br>DCP ...................... 0,075 g | 6,7 ml de dichlorométhane et 1,7 ml de méthanol | 2 heures à 70°C | 140 | 0,16 | 0,17 |
| 12* | lipide 4 ................... 0,713 g<br>cholestérol ............... 0,277 g<br>hexadecanediol 1-2 ..... 0,435 g<br>DCP ...................... 0,075 g | 6,7 ml de dichlorométhane et 1,7 ml de méthanol | 2 heures à 70°C | 181 | 0,15 | 0,65 |

* ne fait pas partie de l'invention

## TABLEAU II (suite 4)

| Exemple N° | Composition de la phase lipidique | Conditions de préparation: Solvant utilisé dans préparation phase lipidique | Conditions de préparation: Agitation du mélange | d (nm) | Q polydispersité | DO450 |
|---|---|---|---|---|---|---|
| 13 | lipide 4 .................. 0,713 g<br>cholestérol .............. 0,277g<br>mélange (phytanetriol +<br>phytanetétrol) .......... 0,435 g<br>DCP ..................... 0,075 g | 5 ml de dichlorométhane et 1,7 ml de méthanol | 2 heures à 70°C | 133 | 0,21 | 0,14 |
| 14* | lipide 1 .................. 1,050 g<br>cholestérol .............. 0,375 g<br>DCP ..................... 0,075 g | 10 ml de dichlorométhane et 2ml de méthanol | 2 heures à 45° C | 137 | 0,22 | 0,39 |
| 15 | lipide 1 .................. 1,050 g<br>phytanetriol ............. 0,375 g<br>DCP ..................... 0,075 g | 10 ml de dichlorométhane et 2ml de méthanol | 2 heures à 45° C | 118 | 0,21 | 0,10 |

* ne fait pas partie de l'invention

Dans ce tableau, les différents constituants sont les suivants :

Lipide 1 : lécithine commercialisée par la société "LUCAS MEYER"sous la dénomination "EPIKURON 200"

Cholestérol : commercialisé par la société "PROLABO"

DCP : dicétylphosphate de sodium

Lipide 2 : lécithine hydrogénée commercialisée par la société "QUEST INTERNATIONAL" sous la

dénomination "LECINOL S 10"

Lipide 3 : lipide amphiphile non-ionique : alcool hexadécylique polyoxyéthyléné contenant en moyenne 20 motifs d'oxyde d'éthylène, commercialisé par la société "ICI ATLAS" sous la dénomination "BRU 58"

Lipide 4 : lipide amphiphile non-ionique de formule :

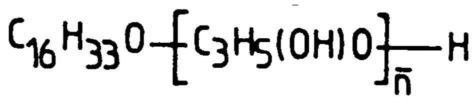

où $-C_3H_5(OH)O-$ est représenté par les structures suivantes prises en mélange ou séparément :

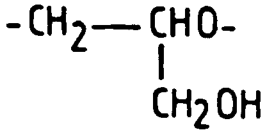

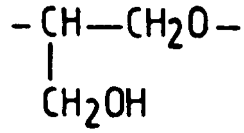

et $\bar{n}$ est une valeur statistique moyenne égale à 3

Mélange (phytanetriol + phytanetétrol) : mélange d'environ 1/1 en poids de phytanetriol et d'un tétrol constitué par les isomères suivants pris en mélange ou séparément :

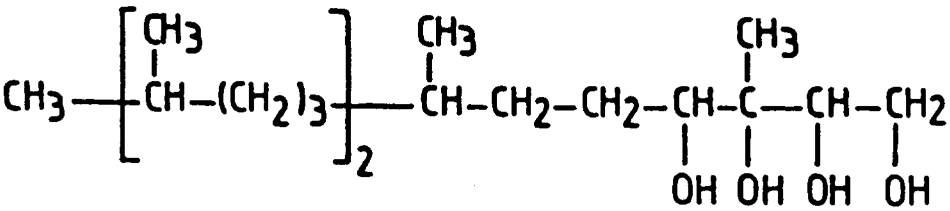

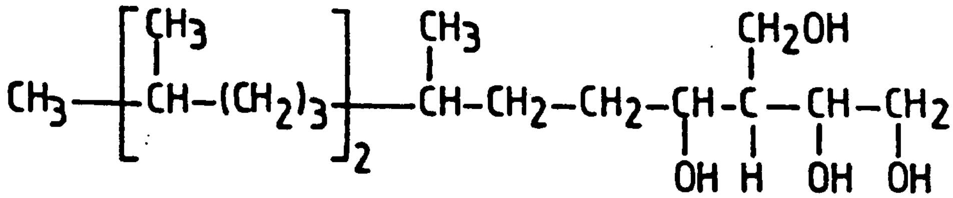

**Revendications**

1. Composition cosmétique et/ou pharmaceutique contenant au moins une dispersion, dans une phase aqueuse de dispersion, de vésicules délimitées par une membrane de phase lipidique contenant au moins un lipide amphiphile ionique et/ou au moins un lipide amphiphile non-ionique, lesdites vésicules contenant une phase encapsulée, caractérisée par le fait que la phase lipidique d'au moins une dispersion contient au moins un composé de formule :

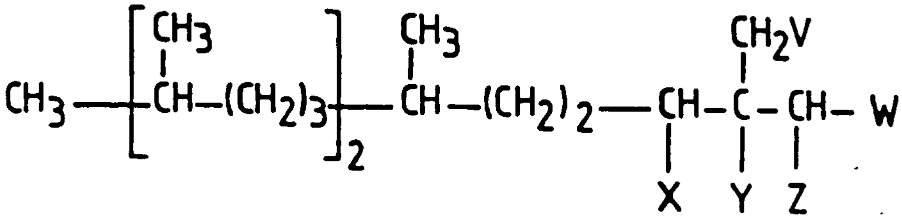 (I)

formule (I) dans laquelle :

- ou bien W représente $-CH_2OH$, -COOM ou $-(CH_2)_2-COOM$, où M représente H, un métal alcalin ou un métal alcalinoterreux, auquel cas X, Y, Z et V, identiques ou différents, représentent un atome d'hydrogène ou un radical hydroxyle, avec la condition que, lorsque W représente $-CH_2OH$, au moins un des radicaux X, Y, Z ou V représente un radical hydroxyle ;
- ou bien W représente un groupement $-CH_3$, auquel cas X, Y, Z et V représentent H ou -OH combinés comme indiqué dans chaque ligne du tableau ci-dessous :

| X | Y | Z | V |
|---|---|---|---|
| OH | OH | OH | OH |
| OH | H | OH | OH |
| OH | OH | OH | H |
| H | OH | OH | OH |
| H | OH | OH | H |
| H | H | OH | OH |
| OH | H | OH | H |

**2.** Composition selon la revendication 1, caractérisée par le fait que le(s) composé(s) de formule (I) est (ou sont) choisi(s) dans le groupe formé par le phytanetriol, l'acide 5,9,13,17-tétraméthyl octadécanoïque, le 3,7,11,15-tétraméthyl 1,2,3,4-tétrahydroxy hexadécane et le 3-hydroxyméthyl 7,11,15-triméthyl 1,2,4-trihydroxy hexadécane.

**3.** Composition selon l'une des revendications 1 ou 2, caractérisée par le fait que le(s) composé(s) de formule (I) représente(nt) 5 % à 90 % en poids de la phase lipidique de la dispersion.

**4.** Composition selon l'une des revendications 1 à 3, caractérisée par le fait que la phase lipidique constitutive des membranes des vésicules comprend au moins un lipide choisi dans le groupe formé par :

A) les lipides non-ioniques ci-après définis :

(1) les dérivés du glycérol, linéaires ou ramifiés, de formule

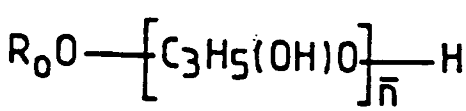 (II)

formule (II) dans laquelle :

- $-C_3H_5(OH)O-$est représenté par les structures suivantes prises en mélange ou séparément : $-CH_2CHOHCH_2O-$,

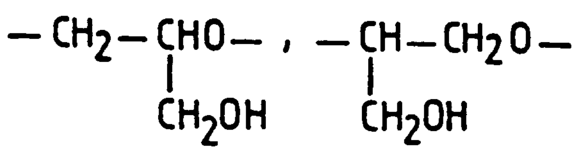

- $\bar{n}$ est une valeur statistique moyenne comprise entre 1 et 6 ou bien n = 1 ou 2 et alors $-C_3H_5(OH)O-$ est représenté par la structure $-CH_2CHOH-CH_2O-$ ;
- $R_o$ représente :
  (a) une chaîne aliphatique, linéaire ou ramifiée saturée ou insaturée, contenant de 12 à 30 atomes de carbone ; ou des radicaux hydrocarbonés des alcools de lanoline ; ou les restes d'alpha-diols à longue chaîne;

(b) un reste $R_1CO$, où $R_1$ est un radical aliphatique, linéaire ou ramifié, en $C_{11}$-$C_{29}$ ;
(c) un reste

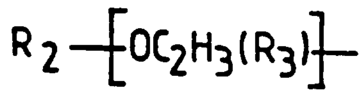

où :
- $R_2$ peut prendre la signification (a) ou (b) donnée pour $R_o$ ;
- $-OC_2H_3(R_3)-$ est représenté par les structures suivantes, prises en mélange ou séparément :

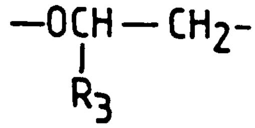

et

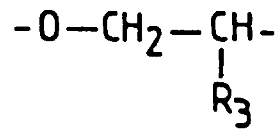

où $R_3$ prend la signification (a) donnée pour $R_o$ ;

(2) les éthers de polyglycérol, linéaires ou ramifiés, comportant deux chaînes grasses ;
(3) les diols à chaîne grasse ;
(4) les alcools gras oxyéthylénés ou non, les stérols et les phytostérols, oxyéthylénés ou non ;
(5) les éthers et esters de polyols, oxyéthylénés ou non, l'enchaînement des oxydes d'éthylène pouvant être linéaire ou cyclique ;
(6) les glycolipides d'origine naturelle ou synthétique, les éthers et esters de mono ou polysaccharides et notamment les éthers et les esters de glucose ;
(7) les hydroxyamides représentés par la formule :

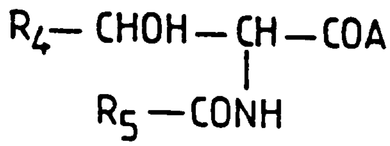

(III)

formule (III) dans laquelle :
- $R_4$ désigne un radical alkyle ou alcényle en $C_7$-$C_{21}$ ;
- $R_5$ désigne un radical hydrocarboné, saturé ou insaturé, en $C_7$-$C_{31}$ ;
- COA désigne un groupement choisi parmi les deux groupements suivants :
  - un reste

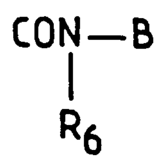

où :
- B est un radical alcoyle dérivé d'amines primaires ou secondaires, mono- ou polyhydroxylées ; et
- $R_6$ désigne un atome d'hydrogène ou un radical méthyle, éthyle ou hydroxyéthyle ; et
- un reste $-COOZ$, où Z représente le reste d'un polyol en $C_3$-$C_7$ ;

(8) les céramides naturels ou de synthèse ;
(9) les dihydroxyalkylamines, les amines grasses oxyéthylénées ;

(10) les dérivés du glycérol répondant à la formule :

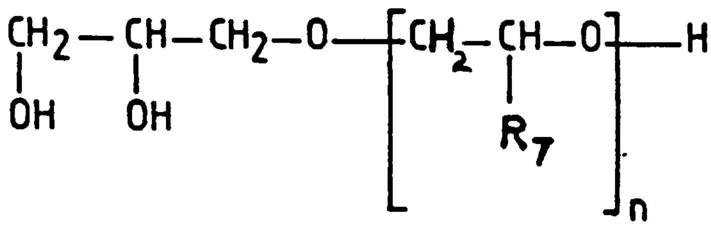

(IV)

formule (IV) dans laquelle $R_7$ représente un radical alkyle linéaire en $C_{14}$ à $C_{18}$ ou un groupement $-CH_2A$ dans lequel A est $-OR_{14}$, $R_{14}$ représentant un radical alkyle linéaire en $C_{10}$-$C_{18}$ et, de préférence, en $C_{16}$, et n représente une valeur statistique moyenne supérieure à 1 et au plus égale à 3 et, en outre, lorsque $R_7 = -CH_2A$, n peut également représenter une valeur réelle (non statistique) égale à 2.

B) les lipides amphiphiles ioniques ci-après définis :

(1) les lipides amphiphiles anioniques suivants :

- les phospholipides naturels, modifiés par voie chimique ou enzymatique, et les phospholipides de synthèse ;
- des composés anioniques, tels que ceux décrits dans le brevet français n° 2 588 256 et représentés par la formule

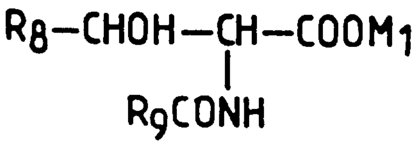

(V)

formule (V) dans laquelle :

- $R_8$ représente un radical alkyle ou alcényle en $C_7$-$C_{21}$,
- $R_9$ représente un radical hydrocarboné, saturé ou insaturé en $C_7$-$C_{31}$, et
- $M_1$ représente H, Na, K, $NH_4$ ou un ion ammonium substitué dérivé d'une amine,

(2) des composés anioniques, constituant le groupe formé par les esters phosphoriques d'alcools gras, l'acide heptylnonylbenzène sulfonique, le sulfate de cholestérol acide et ses sels alcalins, le phosphate de cholestérol acide et ses sels alcalins, les lysolécithines, les alkylsulfates et les gangliosides ;

(3) les lipides amphiphiles cationiques, suivants :

- les composés cationiques dérivés ammonium quaternaire répondant à la formule :

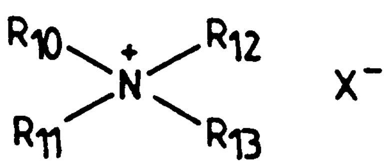

(VI)

avec $R_{10}$ et $R_{11}$, identiques ou différents, représentant des radicaux alkyle en $C_{12}$-$C_{20}$ et $R_{12}$ et $R_{13}$, identiques ou différents, des radicaux alkyle en $C_1$- $C_4$ ;

- les amines à longue chaîne et leurs dérivés ammonium quaternaire, les esters d'aminoalcools à longue chaîne et leurs sels et dérivés ammonium quaternaire ;
- des lipides polymérisables.

**5.** Composition selon l'une des revendications 1 à 4, caractérisée par le fait que la phase aqueuse de dispersion est choisie dans le groupe formé par l'eau et les mélanges d'eau avec au moins un alcool en $C_1$-$C_7$ et/ou un polyol d'alkyle en $C_1$-$C_5$.

**6.** Composition selon l'une des revendications 1 à 5, caractérisée par le fait que la phase lipidique totale de la dispersion représente entre 0,01 % et 50 % en poids par rapport au poids total de la dispersion.

**7.** Composition selon l'une des revendications 1 à 6, caractérisée par le fait que le(s) lipide(s) amphiphile-(s) représente(nt) entre 10 et 95 % en poids par rapport au poids total de la phase lipidique.

**8.** Composition selon la revendication 7, caractérisée par le fait que le(s) lipide(s) amphiphile(s) représente(nt) de 40 à 90 % du poids total de la phase lipidique.

**9.** Composition selon l'une des revendications 1 à 8, caractérisée par le fait que les vésicules ont des dimensions comprises entre 20 et 3 000 nm.

**10.** Composition selon la revendication 9, caractérisée par le fait que les vésicules ont des dimensions comprises entre 20 et 500 nm.

**11.** Composition selon l'une des revendications 1 à 10, caractérisée par le fait qu'elle contient au moins un actif à action cosmétique et/ou pharmaceutique.

**12.** Composition selon l'une des revendications 1 à 11, caractérisée par le fait que la phase lipidique des vésicules de la dispersion contient au moins un composé liposoluble cosmétiquement et/ou pharmaceutiquement actif.

**13.** Composition selon l'une des revendications 11 ou 12, caractérisée par le fait que la phase aqueuse encapsulée contient au moins un composé hydrosoluble cosmétiquement et/ou pharmaceutiquement actif.

**14.** Composition selon l'une des revendications 11 à 13, caractérisée par le fait que la phase aqueuse de dispersion contient au moins un composé hydrosoluble cosmétiquement et/ou pharmaceutiquement actif.

**15.** Composition selon l'une des revendications 11 à 14, caractérisée par le fait qu'elle comprend au moins un additif de formulation assurant sa présentation sous forme de gel, de lotion ou de sérum.

**16.** Procédé de préparation d'une dispersion contenue dans la composition selon l'une des revendications 1 à 15, caractérisé par le fait que :

- on prépare la phase lipidique par dissolution dans un solvant des différents constituants de ladite phase lipidique et on évapore le solvant sous pression réduite ;
- on ajoute la phase aqueuse de dispersion et on homogénéise le mélange par un moyen mécanique et/ou à l'aide d'ultrasons, pour obtenir la dispersion de vésicules.

**17.** Procédé selon la revendication 16, caractérisé par le fait que l'homogénéisation est effectuée à une température comprise entre 10 et 120 °C.

**18.** Dispersion, dans une phase aqueuse de dispersion, de vésicules délimitées par une membrane de phase lipidique contenant au moins un lipide amphiphile ionique et/ou au moins un lipide amphiphile non-ionique, lesdites vésicules contenant une phase encapsulée, caractérisée par le fait que la phase lipidique d'au moins une dispersion contient au moins un composé de formule :

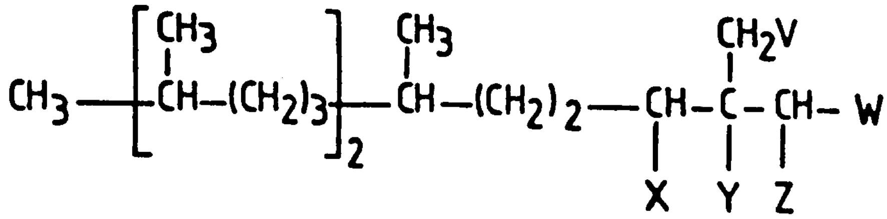

(I)

formule (I) dans laquelle :

- ou bien W représente $-CH_2OH$, $-COOM$ ou $-(CH_2)_2-COOM$, où M représente H, un métal alcalin ou un métal alcalinoterreux, auquel cas X, Y, Z et V, identiques ou différents, représentent un atome d'hydrogène ou un radical hydroxyle, avec la condition que, lorsque W représente $-CH_2OH$ ou COOM, au moins un des radicaux X, Y, Z ou V représente un radical hydroxyle ;
- ou bien W représente un groupement $-CH_3$, auquel cas X, Y, Z et V représentent H ou -OH combinés comme indiqué dans chaque ligne du tableau ci-dessous :

| X | Y | Z | V |
|---|---|---|---|
| OH | OH | OH | OH |
| OH | H | OH | OH |
| OH | OH | OH | H |
| H | OH | OH | OH |
| H | OH | OH | H |
| H | H | OH | OH |
| OH | H | OH | H |

## Claims

1. Cosmetic and/or pharmaceutical composition containing at least one dispersion, in an aqueous dispersion phase, of vesicles delimited by a lipid phase membrane containing at least one ionic amphiphilic lipid and/or at least one nonionic amphiphilic lipid, the said vesicles containing an encapsulated phase, characterized in that the lipid phase of at least one dispersion contains at least one compound of formula:

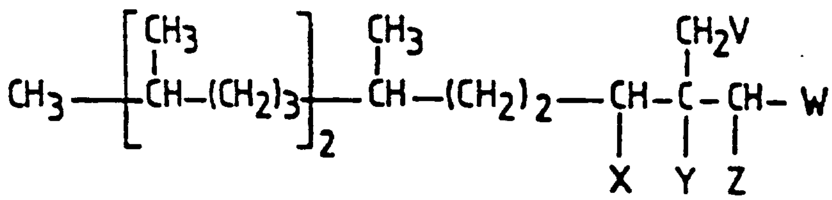

(I)

in which formula (I):

- either W represents $-CH_2OH$, $-COOM$ or $-(CH_2)_2-COOM$, where M represents H, an alkali metal or an alkaline-earth metal, in which case X, Y, Z and V, which are identical or different, represent a hydrogen atom or a hydroxyl radical, with the condition that, when W represents the $-CH_2OH$ group, at least one of the radicals X, Y, Z or V represents a hydroxyl radical;
- or else W represents a $-CH_3$ group, in which case X, Y, Z and V represent H or -OH combined as shown in each line of the table below:

| X | Y | Z | V |
|---|---|---|---|
| OH | OH | OH | OH |
| OH | H | OH | OH |
| OH | OH | OH | H |
| H | OH | OH | OH |
| H | OH | OH | H |
| H | H | OH | OH |
| OH | H | OH | H |

**2.** Composition according to claim 1, characterized in that the compound(s) of formula (I) is (or are) chosen from the group formed by phytanetriol, 5,9,13,17-tetramethyloctadecanoic acid, 3,7,11,15-tetramethyl-1,2,3,4-tetrahydroxyhexadecane and 3-hydroxymethyl-7,11,15-trimethyl-1,2,4-trihydroxyhexadecane.

**3.** Composition according to one of Claims 1 or 2, characterized in that the compound(s) of formula (I) represent(s) 5% to 90% by weight of the lipid phase of the dispersion.

**4.** Composition according to one of Claims 1 to 3, characterized in that the constituent lipid phase of the membranes of the vesicles comprises at least one lipid chosen from the group formed by:
A) the nonionic lipids defined below:
(1) the linear or branched glycerol derivatives of formula

$$R_0O-\left[C_3H_5(OH)O\right]_{\bar{n}}-H \qquad \text{(II)}$$

in which formula (II):

- $-C_3H_5(OH)O-$ is represented by the following structures taken as mixtures or separately : $-CH_2CHOHCH_2O-$ ,

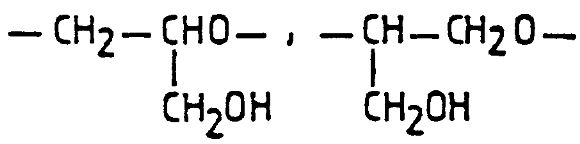

- $\bar{n}$ is a mean statistical value between 1 and 6 or else n = 1 or 2 and then $-C_3H_5(OH)O-$ is represented by the structure $-CH_2CHOH-CH_2O-$;
- $R_o$ represents:
(a) a saturated or unsaturated, linear or branched, aliphatic chain containing from 12 to 30 carbon atoms; or hydrocarbon radicals from lanolin alcohols; or the residues of long-chain alpha-diols;
(b) a residue $R_1CO$, where $R_1$ is a linear or branched, $C_{11}$-$C_{29}$ aliphatic radical;
(c) a residue

$$R_2-\left[OC_2H_3(R_3)\right]-$$

where:

- $R_2$ can take the meaning (a) or (b) given for $R_0$;
- $-OC_2H_3(R_3)-$ is represented by the following structures, taken as a mixture or separately:

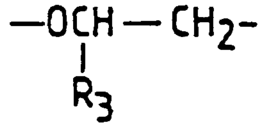 and 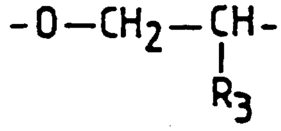

where $R_3$ takes the meaning (a) given for $R_0$;

(2) the linear or branched polyglycerol ethers containing two fatty chains;
(3) the diols containing a fatty chain;
(4) the oxyethylenated or nonoxyethylenated fatty alcohols, or the oxyethylenated or nonoxyethylenated phytosterols or sterols,
(5) the oxyethylenated or nonoxyethylenated ethers and esters of polyols, it being possible for the ethylene oxide chain to be linear or cyclic;
(6) the glycolipids of natural or synthetic origin, the ethers and esters of mono- or polysaccharides and especially the ethers and the esters of glucose;
(7) the hydroxyamides represented by the formula:

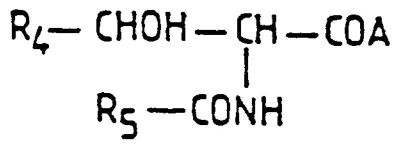 (III)

in which formula (III):

- $R_4$ denotes a $C_7$-$C_{21}$ alkyl or alkenyl radical;
- $R_5$ denotes a saturated or unsaturated $C_7$-$C_{31}$ hydrocarbon radical;
- COA denotes a group chosen from the two following groups:
  - a residue

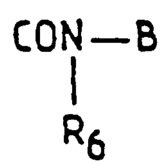

where:

  - B is an alkyl radical derived from mono- or polyhydroxylated, primary or secondary amines; and
  - $R_6$ denotes a hydrogen atom or a methyl, ethyl or hydroxyethyl radical; and
  - a residue -COOZ, where Z represents the residue of a $C_3$-$C_7$ polyol.

(8) the natural or synthetic ceramides;
(9) the oxyethylenated fatty ones or dihydroxyalkylamines;

(10) the glycerol derivatives corresponding to the formula:

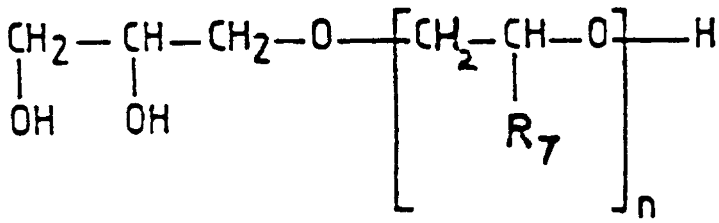

(IV)

in which formula (IV) $R_7$ represents a linear $C_{14}$ to $c_{18}$ alkyl radical or a group $-CH_2A$ in which A is $-OR_{14}$, $R_{14}$ representing a linear $C_{10}$-$C_{18}$ alkyl radical and, preferably, a linear $C_{16}$ alkyl radical, and n represents a mean statistical value greater than 1 and at most equal to 3 and, additionally, when $R_7 = -CH_2A$, n can also represent a true value (non-statistical) equal to 2.

B) the ionic amphiphilic lipids defined below:

(1) the following anionic amphiphilic lipids:

- the natural phospholipids, modified chemically or enzymatically, and the synthetic phospholipids;
- anionic compounds , such as those described in French Patent No. 2,588,256 and represented by the formula

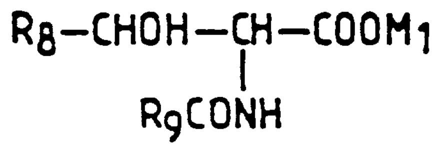

.(V)

in which formula (V):

- $R_8$ represents a $C_7$-$C_{21}$ alkyl or alkenyl radical,
- $R_9$ represents a saturated or unsaturated $C_7$-$C_{31}$ hydrocarbon radical, and
- $M_1$ represents H, Na, K, $NH_4$ or a substituted ammonium ion derived from an amine,

(2) anionic compounds, constituting in the group formed by the phosphoric esters of fatty alcohols, heptylnonylbenzenesulphonic acid, cholesterol acid sulphate and its alkaline salts, cholesterol acid phosphate and its alkaline salts, the lysolecithins, the alkyl sulphates and gangliosides;

(3) the following cationic amphiphilic lipids:

- the quaternary ammonium derived cationic compounds corresponding to the formula:

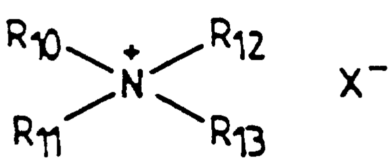

(VI)

with $R_{10}$ and $R_{11}$, which are identical or different, representing $C_{12}$-$C_{20}$ alkyl radicals and $R_{12}$ and $R_{13}$, which are identical or different, $C_1$-$C_4$ alkyl radicals;

- the long-chain amines and their quaternary ammonium derivatives, and the long-chain amino alcohol esters and their salts and quaternary ammonium derivatives;
- polymerizable lipids.

**5.** Composition according to one of Claims 1 to 4, characterized in that the aqueous dispersion phase is chosen from the group formed by water and mixtures of water with at least one $C_1$-$C_7$ alcohol and/or one $C_1$-$C_5$ alkyl polyol.

**6.** Composition according to one of Claims 1 to 5, characterized in that the total lipid phase of the dispersion represents between 0.01% and 50% by weight with respect to the total weight of the dispersion.

**7.** Composition according to one of Claims 1 to 6, characterized in that the amphiphilic lipid(s) represent(s) between 10 and 95% by weight with respect to the total weight of the lipid phase.

**8.** Composition according to Claim 7, characterized in that the amphiphilic lipid(s) represent(s) from 40 to 90% of the total weight of the lipid phase.

**9.** Composition according to one of Claims 1 to 8, characterized in that the vesicles have sizes between 20 and 3000 nm.

**10.** Composition according to Claim 9, characterized in that the vesicles have sizes between 20 and 500 nm.

**11.** Composition according to one of Claims 1 to 10, characterized in that it contains at least one active principle having cosmetic and/or pharmaceutical action.

**12.** Composition according to one of Claims 1 to 11, characterized in that the lipid phase of the vesicles of the dispersion contains at least one cosmetically and/or pharmaceutically active liposoluble compound.

**13.** Composition according to one of Claims 11 or 12, characterized in that the encapsulated aqueous phase contains at least one cosmetically and/or pharmaceutically active water-soluble compound.

**14.** Composition according to one of Claims 11 to 13, characterized in that the aqueous dispersion phase contains at least one cosmetically and/or pharmaceutically active water-soluble compound.

**15.** Composition according to one of Claims 11 to 14, characterized in that it comprises at least one formulation additive providing for its presentation in the gel, lotion or serum form.

**16.** Process for the preparation of a dispersion contained in the composition according to one of Claims 1 to 15, characterized in that :
- the lipid phase is prepared by dissolving various constituents of the said lipid phase in a solvent and the solvent is evaporated under reduced pressure;
- the aqueous dispersion phase is added and the mixture is homogenized by a mechanical means and/or using ultrasound, to produce the vesicle dispersion.

**17.** Process according to Claim 16, characterized in that homogenization is carried out at a temperature between 10 and 120 °C.

**18.** Dispersion, in an aqueous dispersion phase, of vesicles delimited by a lipid phase membrane containing at least one ionic amphiphilic lipid and/or at least one nonionic amphiphilic lipid, the said vesicles containing an encapsulated phase, characterized in that the lipid phase of at least one dispersion contains at least one compound of formula:

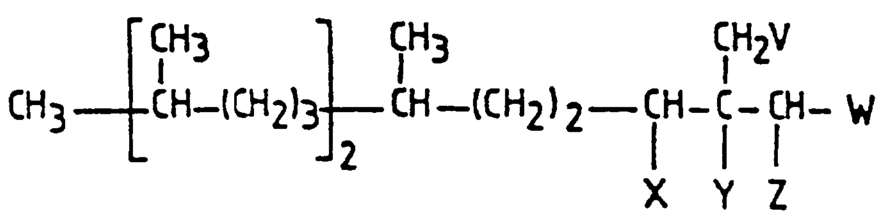

(I)

in which formula (I):
- either W represents $-CH_2OH$, $-COOM$ or $-(CH_2)_2-COOM$, where M represents H, an alkali metal or an alkaline-earth metal, in which case X, Y, Z and V, which are identical or different, represent a

hydrogen atom or a hydroxyl radical, with the condition that, when W represents $-CH_2OH$ or COOM, at least one of the radicals X, Y, Z or V represents a hydroxyl radical;
- or else W represents a $-CH_3$ group, in which case X, Y, Z and V represent H or -OH combined as shown in each line of the table below:

| X | Y | Z | V |
|---|---|---|---|
| OH | OH | OH | OH |
| OH | H | OH | OH |
| OH | OH | OH | H |
| H | OH | OH | OH |
| H | OH | OH | H |
| H | H | OH | OH |
| OH | H | OH | H |

## Patentansprüche

1. Kosmetische und/oder pharmazeutische Zusammensetzung, enthaltend in einer wäßrigen Dispersionsphase wenigstens eine Dispersion von Vesikeln, die durch eine Membran aus einer Lipidphase begrenzt sind, welche wenigstens ein ionisches amphiphiles Lipid und/oder wenigstens ein nichtionisches amphiphiles Lipid enthält, wobei die Vesikel eine Phase eingekapselt enthalten, dadurch gekennzeichnet, daß die Lipidphase der wenigstens einen Dispersion wenigstens eine Verbindung der Formel enthält:

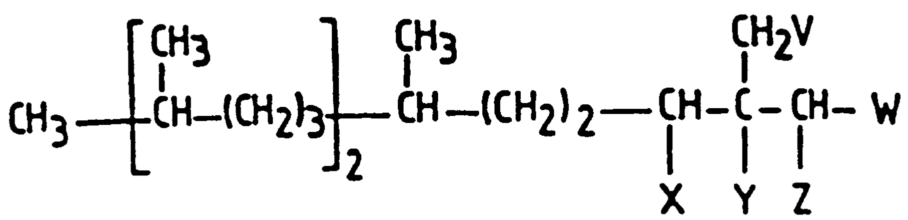
(I)

worin:
- W für $-CH_2OH$, -COOM oder $-(CH_2)_2$-COOM steht, worin M für H, ein Alkalimetall oder ein Erdalkalimetall steht, wobei in diesem Fall X, Y, Z und V, die gleich oder verschieden sind, ein Wasserstoffatom oder eine Hydroxygruppe bedeuten, unter der Bedingung, daß, wenn W für $-CH_2OH$ steht, wenigstens einer der Reste X, Y, Z oder V eine Hydroxygruppe bedeutet;
- oder W für die Gruppe $-CH_3$ steht, wobei in diesem Fall X, Y, Z und V für H oder -OH stehen, die wie in jeder Zeile der nachfolgenden Tabelle angegeben kombiniert sind:

| X | Y | Z | V |
|---|---|---|---|
| OH | OH | OH | OH |
| OH | H | OH | OH |
| OH | OH | OH | H |
| H | OH | OH | OH |
| H | OH | OH | H |
| H | H | OH | OH |
| OH | H | OH | H |

2. Zusammensetzung nach Anspruch 1, dadurch gekennzeichnet, daß die Verbindung (die Verbindungen) der Formel (I) ausgewählt ist (sind) unter Phytantriol, 5,9,13,17-Tetramethyloctadecansäure, 3,7,11,15-Tetramethyl-1,2,3,4-tetrahydroxyhexadecan und 3-Hydroxymethyl-7,11,15-trimethyl-1,2,4-trihydroxyhexadecan.

3. Zusammensetzung nach einem der Ansprüche 1 oder 2, dadurch gekennzeichet, daß die Verbindung (die Verbindungen) der Formel (I) 5 bis 90 Gew.-% der Lipidphase der Dispersion ausmacht (ausmachen).

4. Zusammensetzung nach einem der Ansprüche 1 bis 3, dadurch gekennzeichnet, daß die die Membranen der Vesikel bildende Lipidphase wenigstens ein Lipid umfaßt, das ausgewählt ist unter:

A) den nachfolgend definierten nicht-ionischen Lipiden:

(1) geradkettige oder verzweigte Glycerinderivate der Formel

$$R_0O-[C_3H_5(OH)O]_{\bar{n}}-H \qquad (II)$$

worin:

- $-C_3H_5(OH)O-$
für die folgenden Strukturen in Kombination oder einzeln steht:
$-CH_2CHOHCH_2O-$,

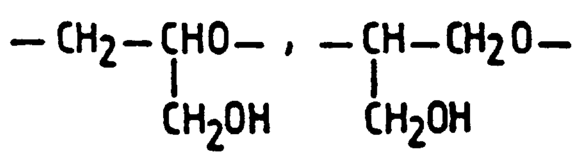

- $\bar{n}$ einen statistischen Mittelwert im Bereich von 1 bis 6 bedeutet oder n = 1 oder 2 und $-C_3H_5(OH)O-$ für die Struktur $-CH_2CHOH-CH_2O-$ steht;
- $R_0$ bedeutet:
(a) eine gesättigte oder ungesättigte, lineare oder verzweigte aliphatische Kette mit 12 bis 30 Kohlenstoffatomen; Kohlenwasserstoffreste von Lanolinalkoholen; oder Reste von langkettigen alpha-Diolen;
(b) einen Rest $R_1CO$, worin $R_1$ einen linearen oder verzweigten aliphatischen $C_{11}$-$C_{29}$-Rest bedeutet;

(c) einen Rest

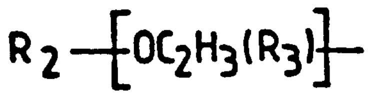

worin:

- $R_2$ die für $R_0$ angegebene Bedeutung (a) oder (b) annehmen kann;
- $OC_2H_3(R_3)$- für die folgenden Strukturen in Kombination oder einzeln steht:

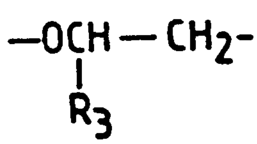 und 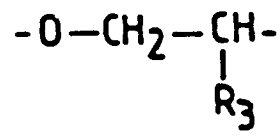

worin $R_3$ die für $R_0$ angegebene Bedeutung (a) hat;

(2) lineare oder verzweigte Polyglycerinether mit zwei Fettketten;

(3) Diole mit einer Fettkette;

(4) ethoxylierte oder nicht-ethoxylierte Fettalkohole, Sterole und Phytosterole, die ethoxyliert oder nicht-ethoxyliert sind;

(5) Ether und Ester von ethoxylierten oder nicht-ethoxylierten Polyolen, wobei die Ethylenoxidkette linear oder cyclisch sein kann;

(6) Glycolipide natürlicher oder synthetischer Herkunft, Ether und Ester von Mono- oder Polysacchariden und insbesondere Glucoseether und -ester.

(7) Hydroxyamide der Formel:

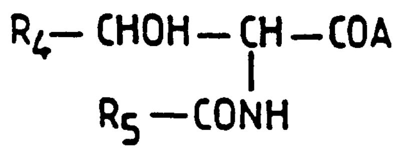 (III)

worin:

- $R_4$ einen $C_7$-$C_{21}$-Alkyl- oder -Alkenylrest bedeutet;
- $R_5$ einen gesättigten oder ungesättigten $C_7$-$C_{31}$-Kohlenwasserstoffrest bedeutet;
- COA für eine Gruppe steht, die ausgewählt ist unter den folgenden Gruppen:
  - einem Rest

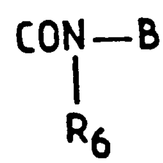

worin:

- B für einen von mono- oder polyhydroxylierten, primären oder sekundären Aminen abgeleiteten Alkylrest steht; und
- $R_6$ ein Wasserstoffatom oder einen Methyl-, Ethyl- oder Hydroxyethylrest bedeutet; und

- einem Rest -COOZ, worin Z den Rest eines $C_3$-$C_7$-Polyols bedeutet;

(8) natürliche oder synthetische Ceramide;

(9) Dihydroxyalkylamine, Fettamine, die ethoxyliert sind;

(10) Glycerinderivate der Formel:

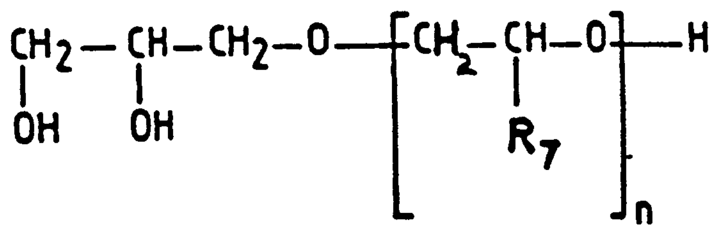

(IV)

worin $R_7$ einen linearen $C_{14}$-$C_{18}$-Alkylrest oder eine Gruppe $-CH_2A$ bedeutet, worin A für $-OR_{14}$ steht, wobei $R_{14}$ einen linearen $C_{10}$-$C_{18}$- und vorzugsweise $C_{16}$-Alkylrest bedeutet und n einen statistischen Mittelwert größer als 1 und höchstens gleich 3 bedeutet und, wenn $R_7 = -CH_2A$, n außerdem auch einen reellen nicht-statistischen Wert von 2 bedeuten kann;

B) den nachfolgend definierten ionischen amphiphilen Lipiden:

(1) folgende anionische amphiphile Lipide:

- natürliche, auf chemischem oder enzymatischem Weg modifizierte Phospholipide und synthetische Phospholipide;
- anionische Verbindungen, wie diejenigen, die im Französischen Patent Nr. 2 588 256 beschrieben sind und die Formel

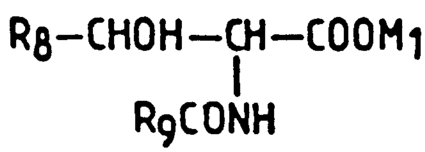

.(V)

besitzen, worin:

- $R_8$ einen $C_7$-$C_{21}$-Alkyl- oder -Alkenylrest bedeutet,
- $R_9$ für einen gesättigten oder ungesättigten $C_7$-$C_{31}$-Kohlenwasserstoffrest steht, und
- $M_1$ für H, Na, K, $NH_4$ oder ein von einem Amin abgeleitetes substituiertes Ammoniumion steht,

(2) anionische Verbindungen der Gruppe, die gebildet wird aus Phosphorsäureestern von Fettalkoholen, Heptylnonylbenzolsulfonsäure, saurem Cholesterinsulfat und seinen Alkalimetallsalzen, saurem Cholesterinphospat und seinen Alkalisalzen, Lysolecithinen, Alkylsulfaten und Gangliosiden;

(3) folgende kationische amphiphile Lipide:

- kationische quaternäre Ammoniumderivate der Formel:

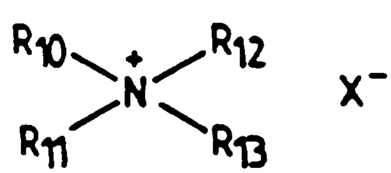

(VI)

wobei $R_{10}$ und $R_{11}$, die gleich oder verschieden sind, $C_{12}$-$C_{20}$-Alkylreste bedeuten und $R_{12}$ und $R_{13}$, die gleich oder verschieden sind, $C_1$-$C_4$-Alkylreste bedeuten;

- langkettige Amine und ihre quaternären Ammoniumderivate, Ester von Aminoalkoholen mit langer Kette und ihre Salze und quaternären Ammoniumderivate;
- polymerisierbare Lipide:

**5.** Zusammensetzung nach einem der Ansprüche 1 bis 4, dadurch gekennzeichnet, daß die wäßrige Phase der Dispersion ausgewählt ist unter Wasser und Gemischen von Wasser mit wenigstens einem $C_1$-$C_7$-Alkohol und/oder $C_1$-$C_5$-Alkylpolyol.

6. Zusammensetzung nach einem der Ansprüche 1 bis 5, dadurch gekennzeichnet, daß die gesamte Lipidphase der Dispersion 0,01 bis 50 Gew.-%, bezogen auf das Gesamtgewicht der Dispersion, ausmacht.

7. Zusammensetzung nach einem der Ansprüche 1 bis 6, dadurch gekennzeichnet, daß das amphiphile Lipid (die amphiphilen Lipide) 10 bis 95 Gew.-%, bezogen auf das Gesamtgewicht der Lipidphase, ausmacht (ausmachen).

8. Zusammensetzung nach Anspruch 7, dadurch gekennzeichnet, daß das amphiphile Lipid (die amphiphilen Lipide) 40 bis 90% des Gesamtgewichtes der Lipidphase ausmacht (ausmachen).

9. Zusammensetzung nach einem der Ansprüche 1 bis 8, dadurch gekennzeichnet, daß die Vesikel Abmessungen im Bereich von 20 bis 3000 nm aufweisen.

10. Zusammensetzung nach Anspruch 9, dadurch gekennzeichnet, daß die Vesikel Abmessungen im Bereich von 20 bis 500 nm aufweisen.

11. Zusammensetzung nach einem der Ansprüche 1 bis 10, dadurch gekennzeichnet, daß sie wenigstens einen Wirkstoff mit kosmetischer und/oder pharmazeutischer Wirkung enthält.

12. Zusammensetzung nach einem der Ansprüche 1 bis 11, dadurch gekennzeichnet, daß die Lipidphase der Vesikel der Dispersion wenigstens eine fettlösliche, kosmetisch und/oder pharmazeutisch aktive Verbindung enthält.

13. Zusammensetzung nach einem der Ansprüche 11 oder 12, dadurch gekennzeichnet, daß die eingekapselte wäßrige Phase wenigstens eine wasserlösliche, kosmetisch und/oder pharmazeutisch aktive Verbindung enthält.

14. Zusammensetzung nach einem der Ansprüche 11 bis 13, dadurch gekennzeichnet, daß die wäßrige Phase der Dispersion wenigstens eine wasserlösliche kosmetisch und/oder pharmazeutisch aktive Verbindung enthält.

15. Zusammensetzung nach einem der Ansprüche 11 bis 14, dadurch gekennzeichnet, daß sie wenigstens ein Formulierungsadditiv enthält, das die Darbietung in Form eines Gels, einer Lotion oder eines Serums ermöglicht.

16. Verfahren zur Herstellung einer in einer Zusammensetzung nach einem der Ansprüche 1 bis 15 enthaltenen Dispersion, dadurch gekennzeichnet, daß man:
    - die Lipidphase herstellt durch Auflösen der unterschiedlichen Bestandteile der Lipidphase in einem Lösungsmittel und das Lösungsmittel unter verringertem Druck verdampft;
    - die wäßrige Phase der Dispersion zugibt und das Gemisch mechanisch und/oder mit Hilfe von Ultraschall homogenisiert, um die Vesikeldispersion zu erhalten.

17. Verfahren nach Anspruch 16, dadurch gekennzeichnet, daß das Homogenisieren bei einer Temperatur im Bereich von 10 bis 120 °C erfolgt.

18. Dispersion von Vesikeln in einer wäßrigen Dispersionsphase, die durch eine Membran aus einer Lipidphase begrenzt sind, welche wenigstens ein ionisches amphiphiles Lipid und/oder wenigstens ein nicht-ionisches amphiphiles Lipid enthält, wobei die Vesikel eine wäßrige Phase eingekapselt enthalten, dadurch gekennzeichnet, daß die Lipidphase der wenigstens einen Dispersion wenigstens eine Verbindung der Formel enthält:

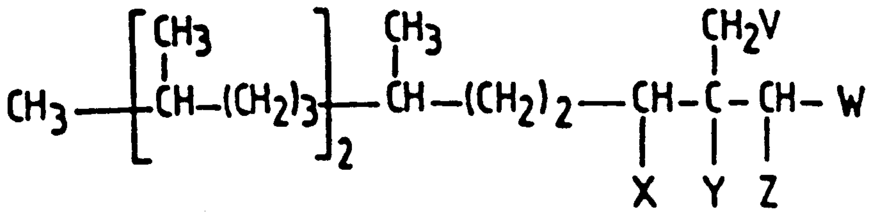

(I)

worin:

- W für $-CH_2OH$, -COOM oder $-(CH_2)_2$-COOM steht, worin M für H, ein Alkalimetall oder ein Erdalkalimetall steht, wobei in diesem Fall X, Y, Z und V, die gleich oder verschieden sind, ein Wasserstoffatom oder eine Hydroxygruppe bedeuten, unter der Bedingung, daß, wenn W für $-CH_2OH$ oder COOM steht, wenigstens einer der Reste X, Y, Z oder V eine Hydroxygruppe bedeutet;
- oder W für die Gruppe $-CH_3$ steht, wobei in diesem Fall X, Y, Z und V für H oder -OH stehen, die wie in jeder Zeile der nachfolgenden Tabelle angegeben kombiniert sind:

| X | Y | Z | V |
|---|---|---|---|
| OH | OH | OH | OH |
| OH | H | OH | OH |
| OH | OH | OH | H |
| H | OH | OH | OH |
| H | OH | OH | H |
| H | H | OH | OH |
| OH | H | OH | H |